# DEMANDE DE BREVET EUROPEEN

(11) **EP 2 549 398 A2**
(43) Date de publication de la demande: **23.01.2013**
(21) Numéro de dépôt: 12177004.4
(22) Date de dépôt: 19.07.2012
(51) Int. Cl.: G06F 19/00

(54) **Système de téléprésence**

(30) Priorité: 19.07.2011 FR 1102234
(71) Demandeur: Ghrenassia, Gilles, 75019 Paris (FR)
(72) Inventeur: Ghrenassia, Gilles, 75019 Paris (FR)
(74) Mandataire: Breesé, Pierre

(57) **Abrégé**

la présente invention concerne un système de téléprésence constitué par un équipement local robotisé et une plateforme de commande à distance, l'équipement robotisé comportant des moyens d'interaction avec un patient caractérisé en ce que l'équipement local est composé de deux sous-ensembles complémentaires, un premier sous-ensemble étant constitué par une station fixe (1) équipée d'un calculateur pour le traitement des commandes provenant de la plateforme de commande d'une part et du second sous-ensemble d'autre part, ainsi que des moyens de dialogue avec d'une part la plateforme de commande à distance, et d'autre part avec un second sous-ensemble mobile (7), ladite station fixe (1) présentant des moyens de couplage mécanique et d'alimentation électrique (16, 17) du sous-ensemble mobile lorsque ce dernier est en position de repos, et des moyens d'échange radiofréquence lorsque le sous-ensemble mobile est activé, ce dernier comportant un second calculateur pour commander les capteurs et actionneurs en fonction desdits échanges radiofréquence avec ladite station fixe.

## Description

### Domaine de l'invention

La présente invention concerne le domaine des équipements de téléprésence, notamment pour des applications de télémédecine et de visio-assistance.

### Etat de la technique

On connaît dans l'état de la technique différents équipements de télémedecine, constitués par un équipement robotisé installé chez un patient, et dialoguant avec une station distante.

En particulier, le brevet US20040019406 de la société IntouchHealth décrit un système comprenant un robot commandé à distance. Le robot est équipé d'une caméra, d'un écran et d'une plateforme holonomique pour le déplacement multidirectionnel. La station de contrôle à distance du robot comprend également une caméra et un moniteur. La station de contrôle distance est reliée au robot par une liaison sans fil. Le robot mobile est destiné à suivre les déplacements du patient et faciliter sa localisation.

La demande de brevet américain US2007061041 décrit un système de navigation robotisé comprenant un robot mobile dans un environnement inconnu. Le robot mobile comprend un serveur internet permettant d'établir un contrôle à partir d'un navigateur web externe. Le robot comprend aussi des moyens pour établir une liaison radiofréquence bidirectionnelle locale à courte portée pour le positionnement par rapport à des repères locaux.

Le brevet US7174238 décrit un système robotisé comprenant un double système de guidage, avec des moyens locaux de faible portée et une commande à distance utilisant le protocole TCP/IP.

La demande de brevet américain US20070192910 décrit un robot mobile de type « compagnon » destiné à assister une personne humaine dans ses déplacements et à assurer une communication selon un mode de téléconférence. Il comporte des moyens de détection et de signalisation des obstacles, ainsi que des moyens de contrôle des données transmises pour assurer le respect des données personnelles.

La demande de brevet américain US20090055023 décrit un système comprenant une station de base et un robot mobile comprenant une caméra et une imprimante. La station de base comporte une interface homme-machine de type graphique pour interagir avec le robot mobile, destiné notamment à la transmission à distance de prescriptions médicales imprimées par l'imprimante du robot.

La demande de brevet américain US20100010672 décrit un système robotisé commandé à distance incluant un robot mobile équipée d'une caméra et d'un système de branchement électrique automatisé pour assurer la recharge des batteries.

Le robot mobile comporte des moyens d'alignement par rapport à une prise électrique fixe.

### Inconvénients de l'état de la technique.

Les solutions proposées dans l'état de la technique impliquent la mise en oeuvre d'un robot complexe, conjuguant des capacités de mobilités avec des capacités d'interaction avec une plateforme à distance, et des moyens d'interactions locaux.

La mise en oeuvre d'un tel équipement est difficile, notamment pour un usage domestique dans un environnement qui n'est pas préparé ni conçu pour faciliter le déplacement d'un équipement robotisé.

### Solution apportée par l'invention

Afin de remédier aux inconvénients de l'état de la technique, l'invention propose selon son acception la plus générale un système de téléprésence constitué par un équipement local robotisé et une plateforme de commande à distance, l'équipement robotisé comportant des moyens d'interaction avec un patient. L'équipement local est composé de deux sous-ensembles complémentaires.

Un premier sous-ensemble est constitué par une station fixe équipes d'un calculateur pour le traitement des commandes provenant de la plateforme de commande d'une part et du second sous-ensemble d'autre part, ainsi que des moyens de dialogue avec d'une part la plateforme de commande à distance, et d'autre part avec un second sous-ensemble mobile. La station fixe présente des moyens de couplage mécanique et d'alimentation électrique du sous-ensemble mobile lorsque ce dernier est en position de repos, et des moyens d'échange radiofréquence lorsque le sous-ensemble mobile est activé, ce dernier comportant un second calculateur pour commander les capteurs et actionneurs en fonction desdits échanges radiofréquence avec ladite station fixe.

L'invention permet ainsi de répartir les ressources de calcul, d'interaction locale et d'interaction distante, et mécaniques de manière optimale. La répartition de ces ressources permet aussi d'améliorer la robustesse mécanique et informatique en réduisant les risques de défaillances se produisant dans des équipements intégrés complexes.

### Variantes de l'invention

Selon une première variante, ledit sous-ensemble mobile comporte un bras articulé équipé d'une pince apte à actionner une poignée de porte.

De préférence, ledit sous-ensemble mobile comporte au moins un microphone et au moins une caméra, ainsi que des moyens de transmission des signaux audiovisuels acquis.

Avantageusement, ledit sous-ensemble mobile comporte au moins un moyen de cartographie volumétrique de l'environnement local ainsi que des moyens de transmission des informations détectées par ledit moyen de cartographie.

Selon une autre variante, ledit sous-ensemble mobile comporte au moins un moyen de communication vocale commandé par la plateforme par l'intermédiaire du sous-ensemble fixe.

Selon un mode de réalisation particulier, le sous-ensemble mobile comporte au moins un moyen de délivrance robotisé.

Selon une variante particulière, le système selon l'invention comporte en outre au moins un moyen portatif d'alerte activable par un usager, ledit moyen d'alerte étant apte à communiquer avec la plateforme par l'intermédiaire de la station fixe.

Selon une autre variante particulière, la station comporte une télécommande de fonctions de télécommunication intégrées dans ladite station.

De préférence la station fixe comporte un logement pour l'intégration du sous-ensemble mobile au repos.

### Description détaillée d'un exemple non limitatif de réalisation

L'invention sera mieux comprise à la lecture de la description se référant à un exemple non limitatif de réalisation, illustré par les dessins annexés où :
- la figure 1 représente une vue schématique du dispositif selon l'invention
- la figure 2 représente une vue schématique du robot mobile selon l'invention

### Présentation générale de l'invention

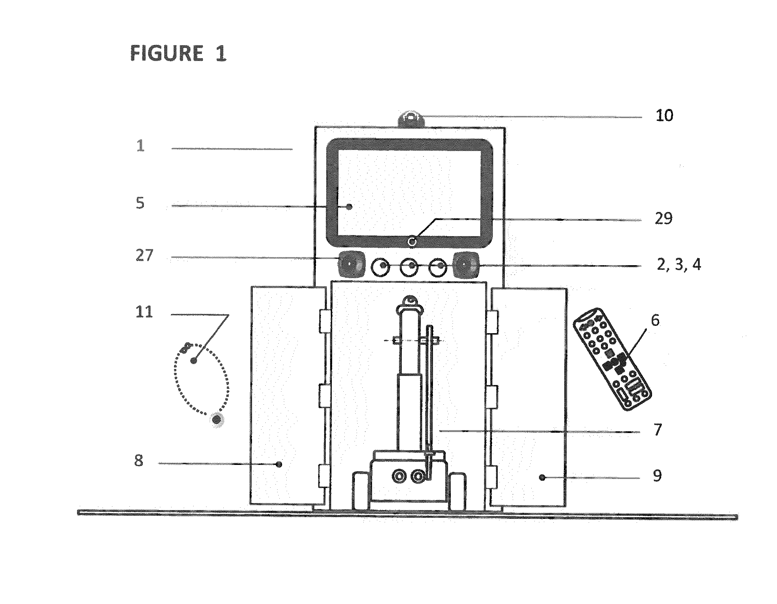

La figure 1 représente une vue schématique du dispositif selon l'invention

Le système selon un exemple de mise en oeuvre de l'invention comprend une station de visioconsultation ainsi qu'un robot mobile.

La station de visio-consultation (1) permet, en présence du médecin généraliste, d'établir une consultation à distance, par exemple sur rendez-vous, entre le patient-abonné et un spécialiste distant. Elle se présente sous la forme d'une borne présentant trois boutons d'appel (2 à 4) accessibles sur la face avant de la station :
- un bouton d'appel (2) vert pour la téléprésence,
- un bouton rouge d'appel (3) pour la téléassistance et
- un bouton blanc d'appel (4) pour la télémédecine.

Elle peut être montée sur roulette pour faciliter son déplacement.

Elle comprend également un écran de visualisation (5). La navigation sur la station multimédia s'effectue grâce à une télécommande infrarouge (6) dédiée.

Les moyens de visio-conférence sont configurés pour permettre l'établissement d'une pluralité de sessions d'urgence simultanées.

La télécommande (6) permet, en dehors des situations d'urgence, d'assurer une liaison audio ou visio avec un interlocuteur quelconque, par exemple un proche ou parent, ou encore un prestataire quelconque, par une session téléphonique ou de visioconférence.

La station présente un logement pour le rangement d'un robot mobile (7). Ce logement est refermable par des portes (8, 9) motorisées.

Une caméra (10) est destinée à engager une téléconférence entre un utilisateur et un opérateur distant. Elle est fixée de manière amovible sur la borne fixe, afin de permettre une utilisation à la main, dans la configuration de session de téléconsultation.

Une alarme se déclenche sur la plateforme si l'alimentation électrique de la station est coupée. Le permanencier appelle l'abonné pour contrôle. Le service informatique peut prendre la main du système informatique, à distance.

En mode veille, l'écran de la station peut servir de cadre photo, d'horloge, de fond d'écran. Des informations locales institutionnelles peuvent être diffusées.

L'auxiliaire peut allumer un projecteur positionné sur le haut de la borne, éclairant ainsi la pièce lors d'une session.

La station (1) comprend des moyens de communication avec le réseau internet, par exemple un liaison ADSL sécurisée pour un accès autonome et sécurisé et avec un accès de secours sur un réseau de communication distinct, par exemple de type 3G ou 4G. En cas de défaillance du réseau principal fixe, les moyens électroniques de la station assurent le transfert automatique de la session sur un autre réseau, par exemple un réseau mobile.

La station (1) comprend par ailleurs un calculateur commandant un routeur pour les échanges bidirectionnels avec le calculateur du robot mobile (7). Elle comprend aussi des moyens d'identification unique de l'équipement. Le calculateur commande par ailleurs une séquence d'initialisation consistant à enregistrer les principales trajectoires et scénarios à effectuer en cas d'urgence.

Cette étape est réalisée localement ou à distance par une série de déplacements commandes par un opérateur, validant ensuite les trajectoires et scénarios appropriés à des situations d'urgence.

Pendant cette séquence d'initialisation, le système enregistre également un plan de l'appartement dans lequel le robot mobile se déplace, pour générer une carte active permettant à l'opérateur distant de situer le robot et d'assurer son déplacement vers un lieu cible préalablement défini sur le plan. La commande de déplacement vers un lieu cible s'effectue par la simple désignation du lieu préalablement enregistré, par exemple par une désignation sur un écran tactile ou la désignation à l'aide d'un pointeur.

Le système comprend en outre un robot mobile.

### Le robot de secours mobile :

La station embarque son propre robot domestique (7) d'assistance vidéo.

En cas d'appel d'urgence, le permanencier actionne la sortie du robot. Commandé par IP, l'unité mobile lui permet de se déplacer virtuellement jusque sur le lieu de l'incident

L'interface multimédia du robot permet à l'opérateur distant de voir, entendre et parler en temps réel avec l'abonné et/ou l'aidant.

Un plan des lieux préalablement établit s'affiche à l'écran du téléopérateur. Un projecteur permet l'éclairage de la trajectoire du robot lors de ses déplacements.

Le robot possède une autonomie de 5 heures en mode vidéo. En fin de session, l'opérateur le reconnecte sur son dock de chargement - au sein de la borne - prêt pour une prochaine utilisation

### La télécommande d'urgence :

L'abonné est en outre équipé de télécommande d'alerte à distance (11) sous forme de pendentif, montre ou clip-ceinture. Grâce à sa télécommande, chaque abonné peut enclencher une communication instantanée avec le plateau de réception d'appels d'un centre de télémédecine.

### Détail du robot mobile

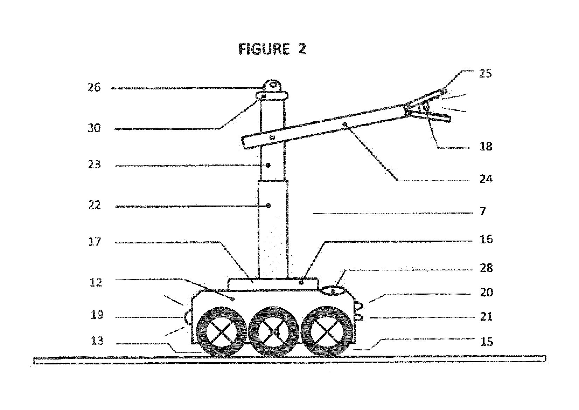

Le robot mobile (7) est représenté plus en détail en figure 2. Il est constitué par une plateforme (11) motorisée équipée de six roues (12 à 14). Cette plateforme présente une embase (12) dans laquelle sont logés une carte de communication radiofréquence, par exemple une carte WIFI (15), ainsi que les circuits électroniques pour le traitement des signaux provenant des capteurs, notamment des capteurs de distance (18, 19) et les signaux transmis aux actionneurs du robot mobile.

L'ensemble des données sont cartographiées lors de la mise en service et traités par un calculateur pour commander localement les déplacements, en fonction des informations transmises par les capteurs (18, 19).

La plateforme (11) comprend également un connecteur (16, 17) pour le branchement sur une prise réseau électrique complémentaire pour le rechargement des batterie électrique lorsque le robot mobile est logé dans la station fixe (1).

La plateforme (11) est surmontée d'un bras mécatronique (19) destiné à interagir mécaniquement avec l'environnement dans lequel le robot se déplace, et notamment à l'actionnement des poignées de porte.

Le bras (19) est constitué par un mat télescopique (20) motorisé pour déplacer verticalement un organe articulé (21) muni à son extrémité d'une pince (22) motorisée.

Le mat télescopique (20) supporte également une caméra mobile (23).

Cet ensemble permet de commander à distance l'ouverture et la fermeture d'une porte ou éventuellement le déplacement d'un obstacle.

Le système comporte en outre un premier haut-parleur (27) positionnés sur la face avant de la borne fixe et un second haut-parleur (28) positionné sur La plateforme du robot mobile. Ces haut-parleur (27, 28) permettent d'assurer le contact audio entre l'opérateur distant et l'utilisateur.

De même, la borne fixe comporte un microphone (29), et le robot mobile un second microphone (30) placé sur le mat mobile (23). Ces microphones permettent d'assurer le contact audio avec l'utilisateur.

Le robot comporte également des moyens de premiers secours, par exemple un équipement de ventilation ou d'oxygénation, ou des médicaments d'urgence qui sont présentés au patient sous le contrôle de l'opérateur distant, commandant le déplacement du robot et la présentation des moyens ou produits d'urgence, ainsi que les instructions d'emploi par l'intermédiaire des moyens de communication du robot mobile. Ces instructions sont transmises sous forme de visioconférence grâce aux moyens audio et vidéo embarqués sur le robot mobile.

## Revendications

1. Système de téléprésence constitué par un équipement local robotisé comportant des moyens d'interaction avec un patient et une plateforme de commande à distance, ledit équipement robotisé local étant composé de deux sous-ensembles complémentaires, un premier sous-ensemble étant constitué par une station fixe (1) équipée d'un calculateur pour le traitement des commandes provenant de la plateforme de commande d'une part et d'un second sous-ensemble mobile (7) d'autre part, ainsi que des moyens de dialogue avec d'une part la plateforme de commande à distance, et d'autre part avec un second sous-ensemble mobile (7),
**caractérisé en ce que** ladite station fixe (1) présentant des moyens de couplage mécanique et d'alimentation électrique (16, 17) du sous-ensemble mobile (7) lorsque ce dernier est en position de repos, et des moyens d'échange radiofréquence lorsque le sous-ensemble mobile (7) est activé, ce dernier comportant un second calculateur pour commander les capteurs et actionneurs en fonction desdits échanges radiofréquence avec ladite station fixe.

2. Système selon la revendication 1 **caractérisé en ce que** ledit sous-ensemble mobile comporte un bras articulé équipé d'une pince apte à actionner une poignée de porte.

3. Système selon la revendication 1 **caractérisé en ce que** ledit sous-ensemble mobile comporte au moins un microphone et au moins une caméra, ainsi que des moyens de transmission des signaux audiovisuels acquis.

4. Système selon la revendication 1 **caractérisé en ce que** ledit sous-ensemble mobile comporte au moins un moyen de cartographie volumétrique de l'environnement local ainsi que des moyens de transmission des informations détectées par ledit moyen de cartographie.

5. Système selon l'une au moins des revendications précédentes **caractérisé en ce que** ledit sous-ensemble mobile comporte au moins un moyen de communication vocale commandé par la plateforme par l'intermédiaire du sous-ensemble fixe.

6. Système selon l'une au moins des revendications précédentes **caractérisé en ce que** ledit sous-ensemble mobile comporte au moins un moyen de délivrance robotisé.

7. Système selon l'une au moins des revendications précédentes **caractérisé en ce qu'**il comporte en outre au moins un moyen portatif d'alerte activable par un usager, ledit moyen d'alerte étant apte à communiquer avec la plateforme par l'intermédiaire de la station fixe.

8. Système selon l'une au moins des revendications précédentes **caractérisé en ce que** la station comporte une télécommande de fonctions de télécommunication intégrées dans ladite station.

9. Système selon l'une au moins des revendications précédentes **caractérisé en ce que** la station comporte un logement pour l'intégration du sous-ensemble mobile au repos.
